# EUROPEAN PATENT APPLICATION

(11) **EP 2 784 707 A2**
(43) Date of publication of application: **01.10.2014**
(21) Application number: 14157012.7
(22) Date of filing: 27.02.2014
(51) Int. Cl.: G06F 19/00

(54) **Portable terminal apparatus and medical image display method**

(30) Priority: 25.03.2013 JP 2013062425
(71) Applicant: Fujifilm Corporation, Minato-ku Tokyo (JP)
(72) Inventor: Okusu, Kiyohisa, Tokyo, 106-8620 (JP); Ueda, Satoshi, Tokyo, 106-8620 (JP); Kudo, Yuya, Tokyo, 106-8620 (JP); Matsumasa, Hironori, Tokyo, 106-8620 (JP); Ohta, Yasunori, Kanagawa (JP)
(74) Representative: Klunker . Schmitt-Nilson . Hirsch

(57) **Abstract**

A portable terminal apparatus for imaging diagnosis includes an object recognition unit (37, 62) for selectively specifying one object (15a) (body part or anatomical region) in a body (15), so as to output object information. A region of the object is changeable according to a terminal position or terminal orientation relative to the body. An image acquisition device (35) acquires a medical image (17) from a server apparatus (32) with relevancy to the object according to the object information. A display unit (22) displays the medical image. Preferably, furthermore, an optical camera optically images the object to create a local image. The object recognition unit specifies the object by image analysis of the local image. Also, the object recognition unit compares the local image with an optical body image created by optically imaging the body wholly in the optical camera, to detect the object in the local image.

## Description

### BACKGROUND OF THE INVENTION

### 1. Field of the Invention

The present invention relates to a portable terminal apparatus and a medical image display method. More particularly, the present invention relates to a portable terminal apparatus and a medical image display method, in which an object in a body of a patient can be specified readily with good handlability.

### 2. Description Related to the Prior Art

An image display apparatus for diagnosis is widely used in a medical field, and displays medical images or diagnostic images, such as X-ray images. In a hospital facility, medical imaging of a body of a patient is carried out, for example, X-ray imaging as radiographic imaging. The medical images obtained by the medical imaging are stored in an image server or server apparatus. The image display apparatus accesses the image server by use of a communication network such as a local area network (LAN), and acquires and displays the medical images.

Various medical imaging apparatus for forming the medical image are known, and include a CT apparatus (computed tomography apparatus) for sectional imaging by use of X-rays, and an MRI apparatus (magnetic resonance imaging apparatus) for sectional imaging by use of magnetic resonance. It has been possible recently to form medical images of a whole body of a patient even in a short time owing to technical development of the CT and MRI apparatuses. In a practical flow of diagnosis, the body is imaged irrespective of his or her lesion. A plurality of the medical images for the body are stored. Then a required one of the medical images with an object (body part or anatomical region) for the diagnosis is read out and displayed on the image display apparatus.

The image server stores combinations of the medical image and object information (body part information or anatomical region information) of the object in the body, such as a head, chest or abdomen. All of the medical images are cumulatively stored in relation to the whole of the body of each of various patients. The image display apparatus, for the purpose of reading out the medical image, transmits a request to the image server together with the object information of a selected one of the objects. The image server responsively distributes or transmits the medical image to the image display apparatus in relation to the object specified by the request.

Various specification methods of specifying the object are known in relation to the image display apparatus. For example, a keyboard or button panel is used for inputting the keywords of the "head, chest or abdomen" to select the object. Also, U.S. Pat. No. 7,769,602 (corresponding to JP-A 2005-296065) discloses a use of a schematic view of the body with divisions of the region in the object of the head, chest or abdomen. In the specification method, a pointer of a mouse is driven to click and select a portion in the schematic view. According to U.S. Pat. No. 7,769,602, it is possible to input command signals more easily and more directly than the specification method in the use of the keyboard.

Furthermore, U.S. Pat.Pub. No. 2009/208,076 (corresponding to JP-A 2009-189541) discloses a portable terminal apparatus for imaging diagnosis including a portable housing and a display panel disposed in front of the housing, in one form of the image display apparatus.

The portable terminal apparatus according to U.S. Pat.Pub. No. 2009/208,076 has the display panel with a smaller size than that of the image display apparatus of an installed type. Although a menu image for the keyboard is displayed for the display panel as required, it is difficult to specify the object by manipulating the keyboard, because the display panel is remarkably small with a small size of the entirety of the keyboard and each alphanumeric elements. It is conceivable to combine the disclosure of U.S. Pat. No. 7,769,602 with the portable terminal apparatus, in which the schematic view of the body is displayed for specifying the object in place of the keyboard. However, the schematic view of the body must have a small size. Low handlability cannot be coped with due to the small size of the display panel.

It is further conceivable to utilize the portable terminal apparatus for oral explanation of a doctor to the patient for findings, diagnosis and treatment of health care service. Complexity in the specification method for specifying the object causes a serious problem because the oral explanation may be interrupted by the use of the portable terminal apparatus with shortcomings.

### SUMMARY OF THE INVENTION

In view of the foregoing problems, an obj ect of the present invention is to provide a portable terminal apparatus and a medical image display method, in which an object in a body of a patient can be specified readily with good handlability.

In order to achieve the above and other objects and advantages of this invention, a portable terminal apparatus for imaging diagnosis includes an object recognition unit for recognizing one object in a body for imaging diagnosis, so as to output object information of the object. An image acquisition device acquires a medical image from an image storage apparatus with relevancy to the object according to the object information. A display unit displays the acquired medical image.

Preferably, the object recognition unit includes an image sampling device for imaging the object in the body to create a local image. An image analyzer analyzes the local image in image analysis, to specify the object for outputting the object information.

Preferably, the image sampling device is an optical camera for creating an optical image. The image analyzer specifies the object by the image analysis of the optical image.

Preferably, the optical camera creates an optical body image of the body and the local image related to the object in the body. The image analyzer specifies the object by comparison between the optical body image and the local image.

Preferably, furthermore, a storage device stores the medical image acquired by the image acquisition device and the local image from the optical camera.

Preferably, the medical image is constituted by plural medical images of two or more imaging modalities different from one another, and/or plural medical images of an imaging modality at two or more image dates different from one another.

Preferably, the image storage apparatus is an image server connected in communication network connection.

In another preferred embodiment, the image sampling device is a thermal camera for creating an image of temperature distribution. The image analyzer specifies the object by the image analysis of the image of the temperature distribution.

Preferably, the thermal camera creates a body thermal image of the body and a local thermal image of the temperature distribution related to the object in the body. The image analyzer specifies the object by comparison between the body thermal image and the local thermal image.

Preferably, furthermore, a storage device stores the medical image acquired by the image acquisition device and the local thermal image from the thermal camera.

Preferably, the medical image is constituted by plural medical images of two or more imaging modalities different from one another, and/or plural medical images of an imaging modality at two or more image dates different from one another.

Preferably, the image storage apparatus is an image server connected in communication network connection.

Also, a medical image display method for a portable terminal apparatus is provided, and includes a step of recognizing one object in a body for imaging diagnosis, so as to output object information of the object. A medical image is acquired from an image storage apparatus with relevancy to the object according to the object information. The acquired medical image is displayed on the portable terminal apparatus.

Preferably, the object recognizing step includes imaging the object in the body with the portable terminal apparatus to create a local image. The local image is analyzed in image analysis, to specify the object for outputting the object information.

Preferably, a computer-executable program for controlling a portable terminal apparatus for imaging diagnosis is provided, and includes a program code for selectively specifying one object in a body, so as to output object information. A region of the object is changeable according to a terminal position or terminal orientation relative to the body. A program code is for acquiring a medical image from an image storage apparatus with relevancy to the obj ect according to the obj ect information. A program code is for displaying the medical image.

Preferably, a user interface for controlling a portable terminal apparatus for imaging diagnosis is provided, and includes a region for selectively specifying one obj ect in a body, so as to output object information. A region of the object is changeable according to a terminal position or terminal orientation relative to the body. A region is for acquiring a medical image from an image storage apparatus with relevancy to the object according to the object information. A region is for displaying the medical image.

Consequently, an object in a body of a patient can be specified readily with good handlability, because a medical image to be acquired remotely is determined according to the object specified in the body by handling the portable terminal apparatus.

### BRIEF DESCRIPTION OF THE DRAWINGS

The above objects and advantages of the present invention will become more apparent from the following detailed description when read in connection with the accompanying drawings, in which:
Fig. 1 is a block diagram schematically illustrating a remote diagnostic system;
Fig. 2A is a perspective view illustrating a portable terminal apparatus;
Fig. 2B is a rear perspective view illustrating the portable terminal apparatus;
Fig. 3 is a block diagram schematically illustrating the portable terminal apparatus;
Fig. 4 is a block diagram schematically illustrating a CPU in the portable terminal apparatus with a server apparatus;
Fig. 5 is an explanatory view illustrating a flow of data for acquisition of a medical image;
Fig. 6A is a flow chart illustrating acquisition of the medical image;
Fig. 6B is a front elevation illustrating imaging of an optical body image;
Fig. 6C is a front elevation illustrating imaging of a local image;
Fig. 6D is a front elevation illustrating display of the medical image;
Fig. 7 is a block diagram schematically illustrating a portable terminal apparatus in another preferred remote diagnostic system;
Fig. 8 is a block diagram schematically illustrating a CPU in the portable terminal apparatus with a server apparatus;
Fig. 9 is an explanatory view illustrating a flow of data for acquisition of a medical image;
Fig. 10A is a flow chart illustrating acquisition of the medical image;
Fig. 10B is a front elevation illustrating imaging of a body thermal image;
Fig. 10C is a front elevation illustrating imaging of a local image;
Fig. 10D is a front elevation illustrating display of the medical image.

### DETAILED DESCRIPTION OF THE PREFERRED EMBODIMENT(S) OF THE PRESENT INVENTION

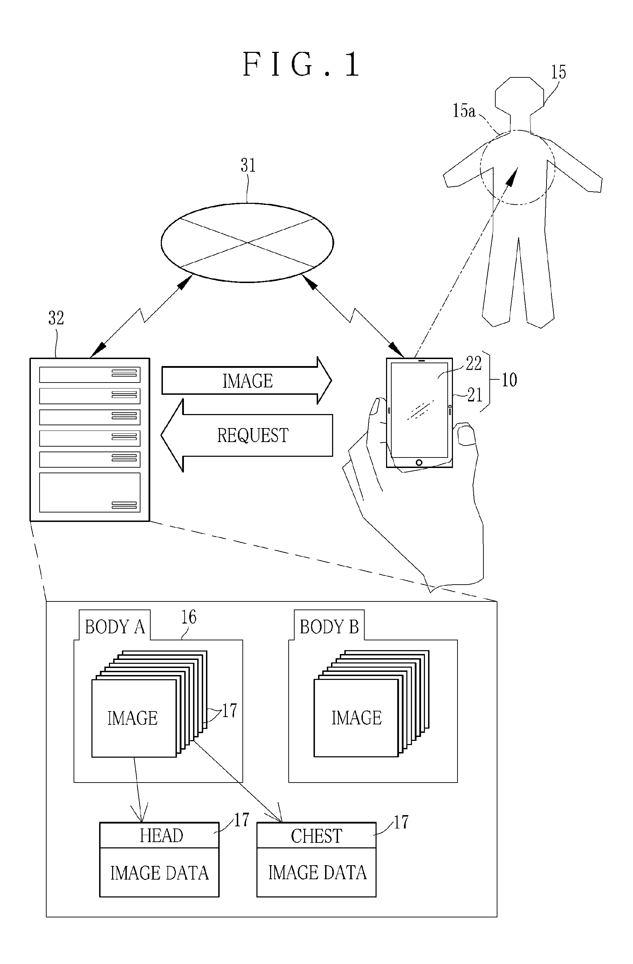

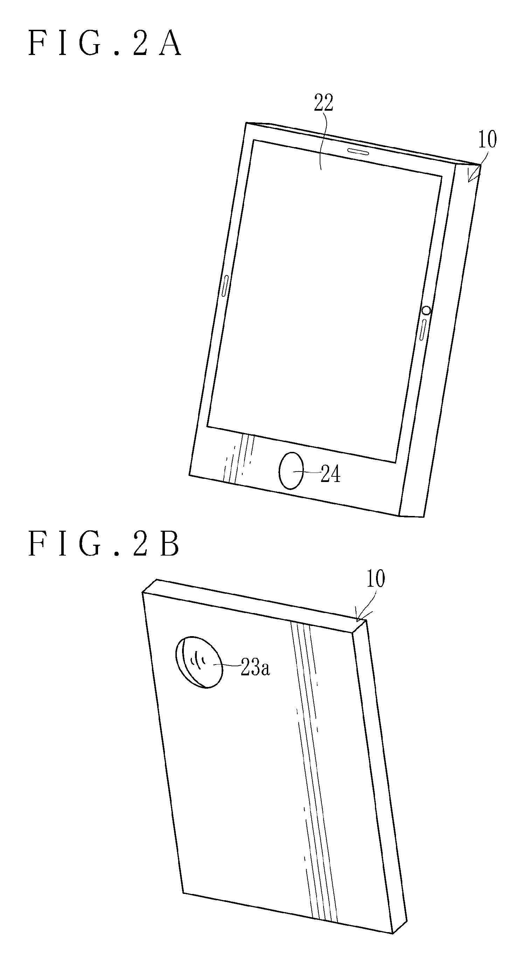

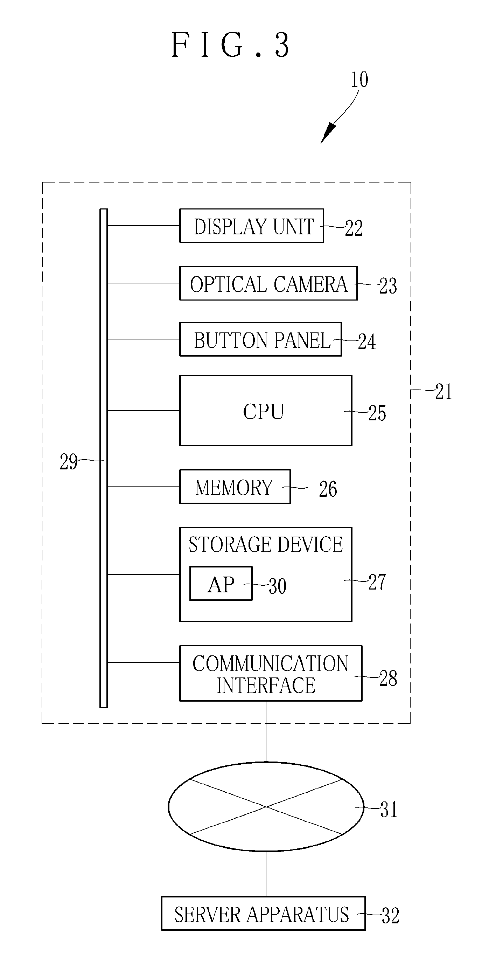

In Fig. 1, a remote diagnostic system is illustrated, and includes a portable terminal apparatus 10 and a server apparatus 32 or image server. The portable terminal apparatus 10 includes a terminal housing 21 of a portable form, and a display unit 22. In Fig. 2A, the display unit 22 is disposed on a front side of the terminal housing 21, and is a liquid crystal panel of a touch panel structure. In Fig. 2B, an optical camera 23 for image sampling of Fig. 3 is incorporated in the terminal housing 21. A lens system 23a or taking lens is disposed on the rear side of the terminal housing 21 as optics in the optical camera 23.

The server apparatus 32 or image server as image storage apparatus is connected with the portable terminal apparatus 10 communicably by a communication network 31, such as a local area network (LAN) in the hospital facility. Medical images 17 or diagnostic images of a body 15 of a patient are remotely distributed from the server apparatus 32 upon access from the portable terminal apparatus 10, so that the display unit 22 of the portable terminal apparatus 10 displays one of the medical images 17. The server apparatus 32 stores the medical images 17 created by a medical imaging apparatus in a manner sorted by a patient ID (patients A and B and the like) as attribute information for recognition. An example of the medical imaging apparatus is a CT apparatus (computed tomography apparatus). The medical images 17 are sectional images. For example, the CT apparatus images the whole of the body 15 in one sequence of imaging, to create an image group 16 or image series including a set of the plurality of the medical images 17 in relation to the body 15. Also, object information is associated with each of the medical images 17 besides image data, and expresses objects such as a head, chest and abdomen of the body 15.

The server apparatus 32 is provided by the portable terminal apparatus 10 with information of a request, which includes the patient ID for recognition of the patient, and object information of an object 15a (body part or anatomical region) in the body 15. According to the object information included in the request, the server apparatus 32 searches and reads the medical image 17 according to the object 15a specified with the object information from the image group 16 of the body 15. The server apparatus 32 transmits the medical image 17 to the portable terminal apparatus 10.

In Fig. 3, the portable terminal apparatus 10 is constituted by installing an application program (AP) 30 in a communication apparatus or electronic apparatus. Examples of the application program 30 are an operating system (OS) and a control program for functioning the communication apparatus as a display apparatus. Examples of the communication apparatus are a cellular telephone, smart phone and the like.

The terminal housing 21 of the portable terminal apparatus 10 includes not only the display unit 22 and the optical camera 23 but a button panel 24 with operation buttons, a CPU 25 as display control device, a memory 26, a storage device 27 and a communication interface 28. A data bus 29 interconnects those elements together with the display unit 22 and the optical camera 23.

The storage device 27 is internal storage incorporated in the terminal housing 21. Examples of the storage device 27 are a hard disk drive (HDD), semiconductor memory and the like. The storage device 27 stores various data, for example, stores a control program and the application program 30 such as software for a console unit.

The application program 30 includes a reading application for displaying personal information and medical chart information (document or text), and an image viewing application for displaying the medical images 17. The personal information has a patient name, patient ID and the like. The chart information includes a medical chart of the patient. The image viewing application is an application for remotely acquiring the medical images 17 from the server apparatus 32 and displaying the medical images 17 on the display unit 22. As will be described later, the image viewing application includes a program for the CPU 25 to execute in an object specification mode (body part recognition mode). The object specification mode is a mode for easily inputting the object specification from a doctor or user of the portable terminal apparatus 10. The chart information is acquired from a chart server apparatus (not shown) through the communication network 31. Thus, the chart information is input to the portable terminal apparatus 10 inclusive of the patient ID. In case the image viewing application or reading application for medical charts is started up, information of an operation menu is displayed on the display unit 22.

The memory 26 is a working memory with which the CPU 25 performs tasks. The CPU 25 loads the memory 26 with the control program read from the storage device 27, and controls various elements in the computer by processing according to the control program. The CPU 25 operates as a display control unit for controlling the display unit 22. The communication interface 28 is a network interface for controlling transmission to the communication network 31, such as a local area network (LAN).

The optical camera 23 includes the lens system 23a and an imaging unit for forming a body image with visible light focused by the lens system 23a. Examples of the imaging unit include a CCD (charge coupled device) image sensor, CMOS (complementary metal oxide semiconductor) image sensor, and the like.

While the imaging mode is set, the optical camera 23 repeatedly performs live imaging in which a live view image is created at a predetermined interval. The live view image formed by the live imaging is displayed on the display unit 22 in a real-time manner. In the imaging mode, the button panel 24 operates as a release button. Upon manipulation of the button panel 24, still imaging is started. The optical camera 23 forms a still image focused by the lens system, and outputs the still image to the storage device 27. The still image is an optical image of which the number of pixels is higher than that of the live view image.

The optical camera 23 for image sampling is utilized for inputting object specification upon startup of the image viewing application. In case the object specification mode (body part recognition mode) is set by starting up the image viewing application, an imaging mode is set to start the optical camera 23 for the live imaging. As the lens system 23a of the optical camera 23 is disposed in the terminal housing 21, an object to be imaged by the optical camera 23 is changed according to a change in the relative position between the terminal housing 21 and the body 15, namely, according to a position of directing the terminal housing 21 to a region in the body 15. In case a still image is created by directing the lens system 23a of the terminal housing 21 to a region in the body 15, for example, the chest, then an optical image of the chest is written to the storage device 27. The portable terminal apparatus 10 automatically recognizes the chest included in the created optical image according to the optical image stored in the storage device 27.

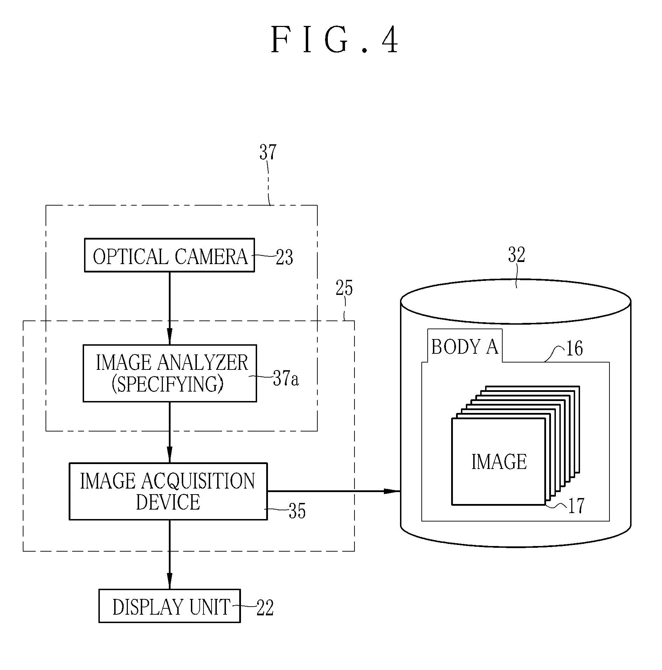

In case an image viewing application included in the application program 30 is started up as illustrated in Fig. 4, the CPU 25 comes to include an image acquisition device 35, and an image analyzer 37a. The optical camera 23 and the image analyzer 37a are combined to constitute an object recognition unit 37. An optical local image 42 is analyzed by the image analyzer 37a, which recognizes an object imaged in the optical local image 42, and outputs object information of the object to the image acquisition device 35. The image acquisition device 35 creates a request of distribution according to the object information from the image analyzer 37a, and sends the request to the server apparatus 32.

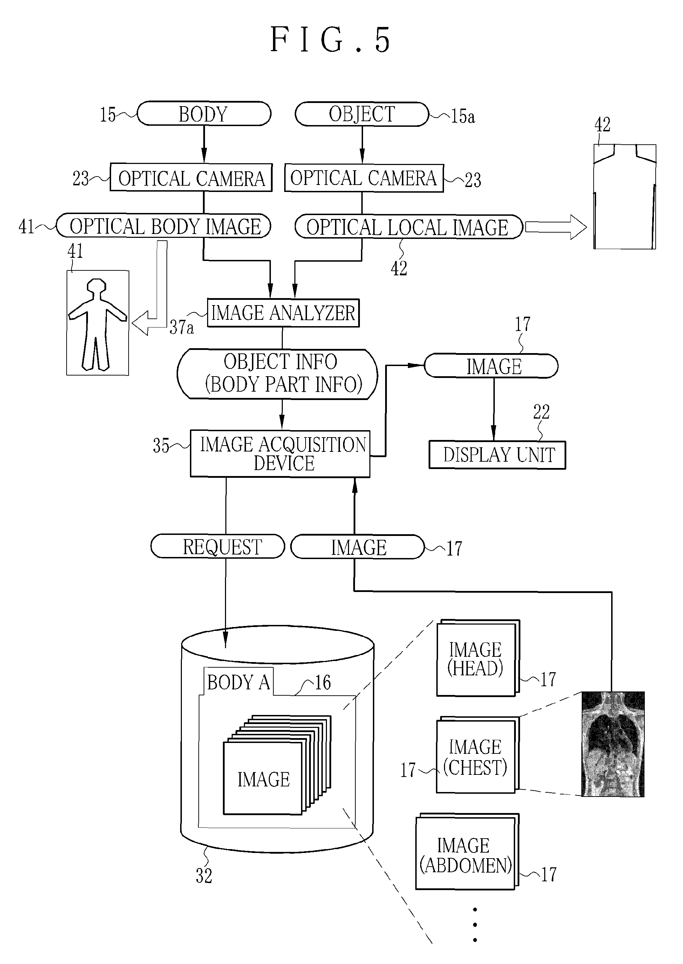

At first, the optical camera 23 operates in the object specification mode (body part recognition mode) as illustrated in Fig. 5. Still images including an optical body image 41 of the whole of the body 15 and the optical local image 42 of the object 15a required by a doctor are formed. Then the optical body image 41 and the optical local image 42 are written to the storage device 27. The image analyzer 37a reads the optical body image 41 and the optical local image 42 from the storage device 27. Then the image analyzer 37a processes the optical body image 41 and the optical local image 42 for extraction of the profiles, and compares the profiles with one another.

The image analyzer 37a carries out the processing for the checking the profile, and extracts a region where a profile in the optical local image 42 coincides with a profile in the optical body image 41. Data for the image viewing application include reference data expressing relationships of various profile lines with the head, chest, abdomen, lower abdomen, arms, legs and the like as objects. Although a size of the body is different between patients, the shapes of the head, chest, abdomen, lower abdomen, arms, legs and the like are common. Therefore, the reference data can be prepared suitably.

The image analyzer 37a refers to the reference data, and detects one of the objects in the optical body image 41 with which the profile in the optical local image 42 coincides. In this manner, the image analyzer 37a carries out the object specification. The image analyzer 37a outputs a result of the object specification to the image acquisition device 35 as object information.

As an object to be imaged by the optical camera 23 is changed according to a change in the relative position of the terminal housing 21 to the body 15, the optical local image 42 to be input to the image analyzer 37a is changed. In response to this, the object recognized by the image analyzer 37a is changed.

The image acquisition device 35 transmits a request to the server apparatus 32 inclusive of the object information, and remotely acquires the medical image 17 relevant to the object specified with the object information from the server apparatus 32. The medical image 17 being acquired is displayed on the display unit 22.

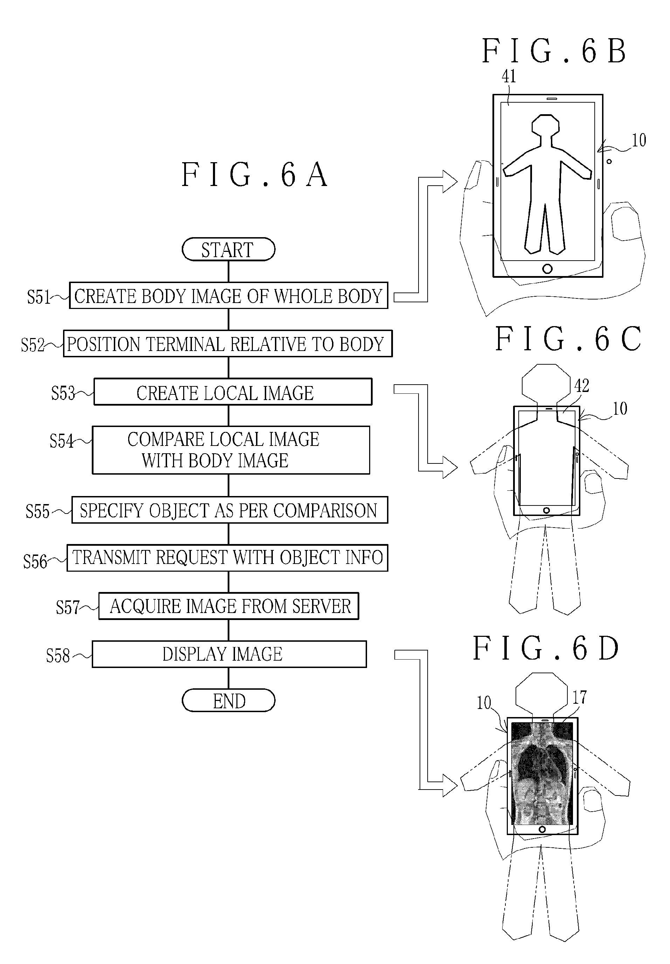

The operation of the above construction is described by referring to a flow chart in Figs. 6A-6D. A doctor or operator uses the portable terminal apparatus 10 in case a patient sees him or her for consultation. To explain findings, symptoms and treatment of the body 15, the doctor views the medical image 17 as a result of imaging of the body 15. The doctor inputs information of a selected object (body part or anatomical region) for the portable terminal apparatus 10 to display one of the medical images 17 relevant to the symptoms of the body 15.

In case the image viewing application is started up, the object specification mode (body part recognition mode) is set. The portable terminal apparatus 10 starts live imaging with the optical camera 23. The doctor views a live view image on the display unit 22 while the lens system 23a of the terminal housing 21 is directed to the body 15, and positions the terminal housing 21 to frame the entirety of the body 15. The button panel 24 is manipulated to form the optical body image 41 (step S51 and Fig. 6B). The optical body image 41 is written to the storage device 27. Then the terminal housing 21 is positioned and directed to the object 15a in the body 15 in relation to the request of transmission (step S52). Assuming that the object 15a is a chest, the terminal housing 21 is directed to the chest. The doctor checks the live view image of the chest on the display unit 22, and manipulates the button panel 24. Consequently, the optical local image 42 as a still image of the chest is created and written to the storage device 27 (step S53 and Fig. 6C).

The image analyzer 37a compares the optical body image 41 with the optical local image 42 after outputting to the storage device 27 (step S54), and detects an object in the optical local image 42 according to a result of the comparison to specify the object (step S55). The image analyzer 37a outputs the object information to the image acquisition device 35. Thus, the inputting operation for the object specification is carried out.

The image acquisition device 35 transmits the request to the server apparatus 32 (step S56), the request including the object information and patient ID. The server apparatus 32 searches and reads the medical image 17 relevant to an object coinciding with an object of the object information according to the request, and transmits the medical image 17 to the portable terminal apparatus 10. The portable terminal apparatus 10 upon remotely receiving the medical image 17 (step S57) drives the display unit 22 to display the medical image 17 (step S58 and Fig. 6D).

The portable terminal apparatus 10 can input signals for object specification only by imaging the whole body 15 and by imaging the object 15a in the body 15 relevant to requirement of data transmission in a suitable orientation of the portable terminal apparatus 10. It is unnecessary to manipulate an user input field or keyboard with the display unit 22 with a display image of a shape of a human body as disclosed in U.S. Pat.Pub. No. 2009/208,076 (corresponding to JP-A 2009-189541). As the display unit 22 of the portable terminal apparatus 10 has a small size of the display image, the display unit 22 is difficult to manipulate because of the small user input field. However, it is possible in the invention to keep high operability because of no requirement of a small user input field.

Assuming that a doctor orally explains diagnosis or treatment to the patient and also manually selects an object by use of the small terminal apparatus with the user input field, difficulty arises in the manipulation and may interrupt the explanation. However, it is possible in the invention to select an object only by positioning the portable terminal apparatus 10 relative to the body 15. The oral explanation can be carried out smoothly without interruption. Also, good handlability with easy access can be obtained in comparison with the operation with a user input field of the schematic view of a human body as disclosed in U.S. Pat. Pub. No. 2009/208,076, because the portable terminal apparatus 10 can be readily positioned relative to an object for the explanation.

It is possible according to this manipulating method for the body 15 to recognize a specific object in the medical image 17 to be displayed by use of the terminal position of the portable terminal apparatus 10. Thus, the object imaged in the medical image 17 can be clarified distinctly. Explanation of a doctor with the medical image 17 can be easily understood by a patient upon medical notification of diagnostic information.

In the present embodiment, the optical body image 41 is formed in addition to the optical local image 42, so that an object in the optical local image 42 is detected by comparison between the optical local image 42 and the optical body image 41. However, the optical body image 41 may not be formed. As described heretofore, there are common features in a profile line of a human body irrespective of minor differences between individuals. Thus, it is possible to detect an obj ect in the optical local image 42 by predetermining reference data for the optical local image 42 as a pattern of a profile of a whole body.

However, precision in the detection is higher in the comparison with the optical body image 41 of a patient than in the comparison with the pattern of a reference profile of a whole body. For example, in the case of imaging the body 15 with his or her clothes, a shape, pattern or the like of the clothes influences a detected profile. In view of this, the optical body image 41 is utilized to facilitate comparison of the optical body image 41 with the optical local image 42, because the shape, pattern or the like of the clothes is also imaged in the optical body image 41 and in the optical local image 42. The precision in the detection can be high. Therefore, it is preferable to form the optical body image 41.

The information of the relative position between the terminal housing 21 of the portable terminal apparatus 10 and an object in the body 15 also includes information of a change of a terminal orientation of the portable terminal apparatus 10 relative to the body 15 as well as a relative height of the portable terminal apparatus 10 to the body 15. For example, the terminal housing 21 can be inclined while the height of the portable terminal apparatus 10 is unchanged, to change a direction of the lens system 23a. Thus, an imaging area can be changed. Specifically, the terminal orientation of the terminal housing 21 is directed downwards by positioning the portable terminal apparatus 10 at a height of a head of the body 15. It is possible to direct the lens system 23a to a chest of the body 15.

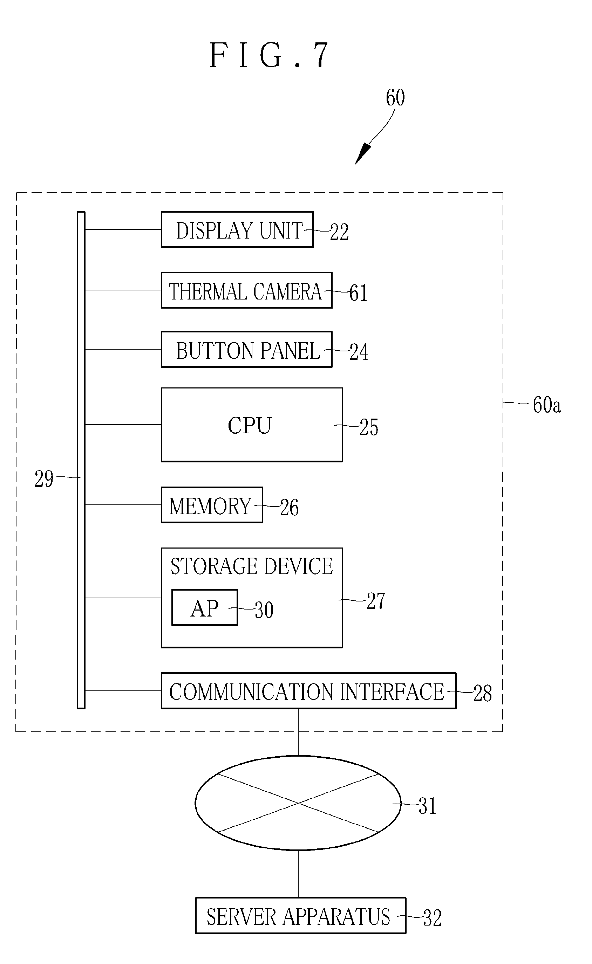

A second preferred embodiment is described by referring to Figs. 7-10D. In a portable terminal apparatus 60 of Fig. 7, the portable terminal apparatus 10 of the first embodiment is repeated but with a difference in which a thermal camera 61 or thermosensor camera is used for image sampling instead of the optical camera 23. Elements similar to those of the above embodiment are designated with identical reference numerals.

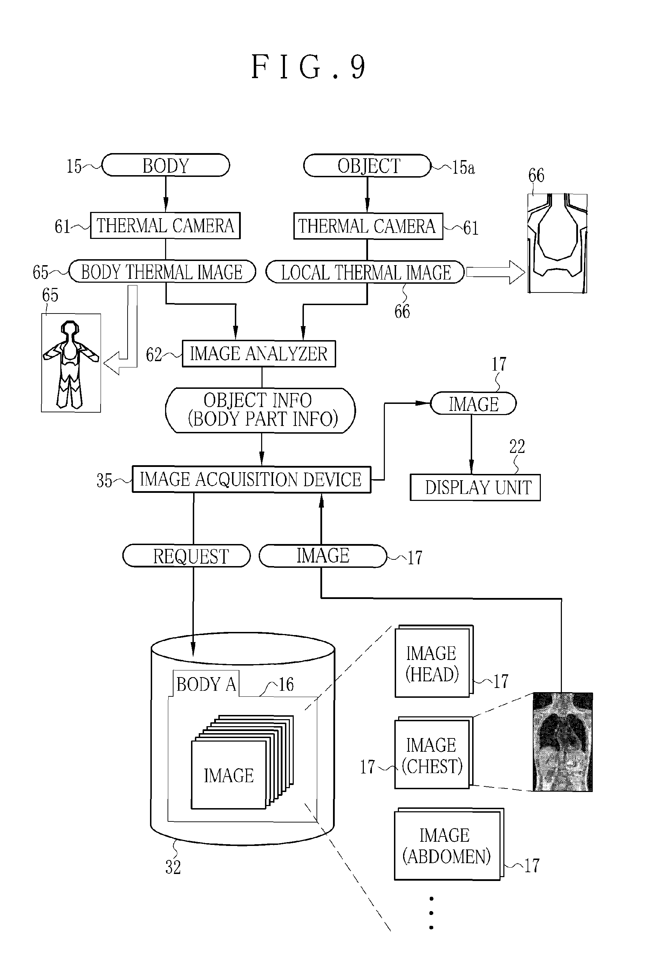

The thermal camera 61 is a camera for forming an image by visualizing temperature distribution in an imaging area. The portable terminal apparatus 60 has a terminal housing 60a. The thermal camera 61 has temperature sensors (not shown), which corresponds to the lens system 23a of the optical camera 23 and disposed on the portable terminal apparatus 60 in a similar manner to the lens system 23a. It is medically known that temperature of a human body is not uniform but different between various objects (body parts or anatomical regions). According to a known temperature distribution, temperature of a head and chest is relatively high. Temperature of an abdomen, hands and feet is relatively low. Thus, it is possible to specify an object of a local thermal image 66 by comparison of a body thermal image 65 and the local thermal image 66 in image analysis for temperature distribution in Fig. 9.

The temperature distribution in each of the thermal images is expressed with a red color for a relatively high temperature, and with colors or orange, yellow, green and blue for lower temperature decreasing in this sequence. In the images of the embodiment, the head and the chest are in the red color. Objects continuing from those are in the colors coming near to blue in a sequence toward distal ends of the abdomen, hands and feet.

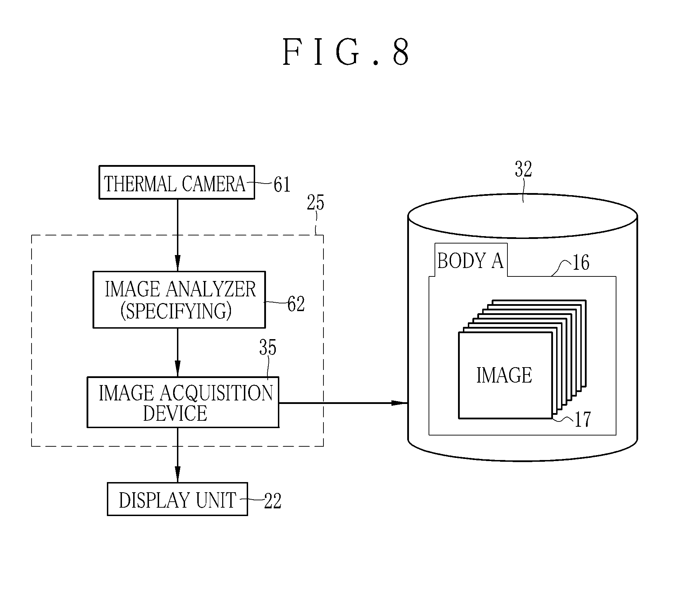

In case the application program 30 is started up, the CPU 25 in the portable terminal apparatus 60 operates with an image analyzer 62 in the object recognition unit, and the image acquisition device 35. See Fig. 8. The image analyzer 62 extracts patterns of temperature distributions of the body thermal image 65 and the local thermal image 66 (see Fig. 9) according to the image analysis, the body thermal image 65 being formed from the whole of the body 15, the local thermal image 66 being formed from the object 15a in the body 15. The distribution pattern from the local thermal image 66 is compared with that from the body thermal image 65, to detect an object included in the local thermal image 66. Then object information is obtained from the result of the comparison.

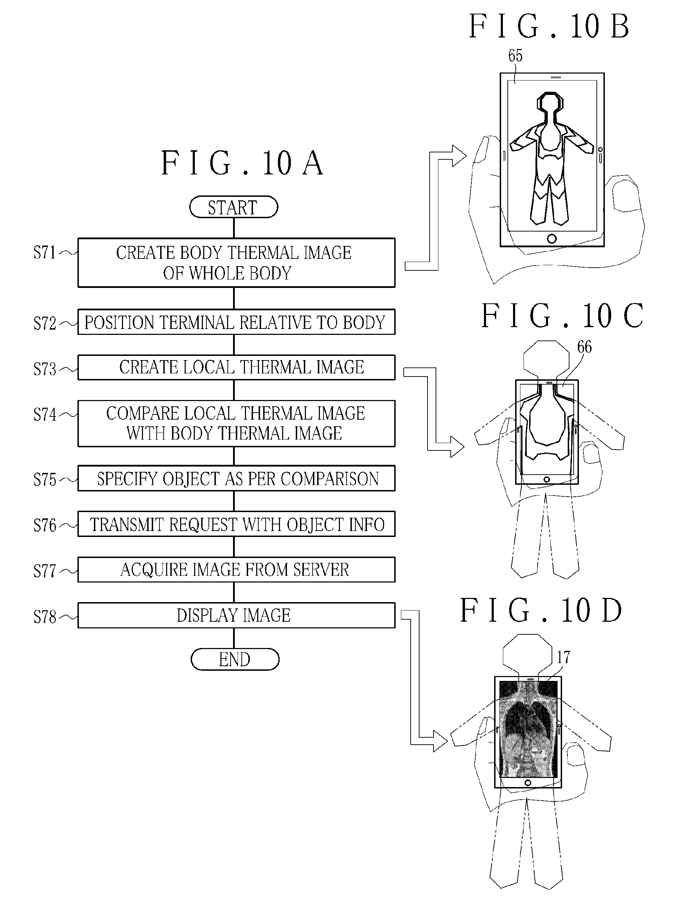

The operation of the above construction is described by referring to the flow chart in Figs. 10A-10D. The portable terminal apparatus 60 is used for a doctor or operator in the health care service of the body 15 in a similar manner to the portable terminal apparatus 10. He or she inputs information for object specification to drive the portable terminal apparatus 60 for displaying the medical image 17 of the object related to the diagnosis or treatment.

In case the object specification mode (body part recognition mode) is set by starting up the image viewing application, the portable terminal apparatus 60 starts live imaging with the thermal camera 61. The doctor directs the terminal housing 60a to the body 15, observes the live view image on the display unit 22, positions the terminal housing 21 in a manner framing the whole of the body 15, and manipulates the button panel 24 to form the body thermal image 65 (step S71 and Fig. 10B). The body thermal image 65 is written to the storage device 27. Then the terminal housing 21 is directed to a region of the object 15a in the body 15 of which the doctor wishes to receive a medical image (step S72). For example, the terminal housing 21 is directed to the chest which is the object 15a. He or she manipulates the button panel 24 after checking the live view image of the chest on the display unit 22. A still image of the chest is formed by way of the local thermal image 66, which is written to the storage device 27 (step S73 and Fig. 10C).

The image analyzer 62 compares the body thermal image 65 with the local thermal image 66 after outputting to the storage device 27 (step S74), and recognizes the object within the local thermal image 66 according to a result of the comparison (step S75). The image analyzer 62 outputs object information to the image acquisition device 35. Thus, the inputting operation for the object specification is carried out.

The image acquisition device 35 transmits the request to the server apparatus 32 (step S76), the request including the object information and patient ID. The server apparatus 32 searches and reads the medical image 17 relevant to an object coinciding with that of the object information according to the request, and transmits the medical image 17 to the portable terminal apparatus 60. The portable terminal apparatus 60 upon remotely receiving the medical image 17 (step S77) drives the display unit 22 to display the medical image 17 (step S78 and Fig. 10D).

Also, in the second embodiment, the object imaged by the thermal camera 61 is changed with a change in the relative position (terminal position or terminal orientation) of the terminal housing 60a of the portable terminal apparatus 60 to the body 15. Responsively, the local thermal image 66 for input to the image analyzer 62 is changed. The object recognized by the image analyzer 62 is changed.

In the second embodiment, the body thermal image 65 may not be formed in a manner similar to the first embodiment. For this situation, the temperature distribution image of a standard distribution of a human body is used for comparison with the body thermal image 65. However, some specificity occurs in the temperature distribution between individuals. It is preferable to form the body thermal image 65 for comparison even in the second embodiment for the purpose of higher precision in the detection in a manner similar to the first embodiment.

In each of the above embodiments, it is possible to keep the medical image 17 stored in the storage device 27 without deletion after the display. It is unnecessary to acquire the medical image 17 from the server apparatus 32 again for the purpose of redisplay, because of the stored condition in the storage device 27. The redisplay of the medical image 17 can be carried out rapidly, because no object specification is required.

Furthermore, the optical local image or local thermal image can be stored in the storage device 27 or storage medium in the terminal apparatus as images obtained in the object specification in addition to the medical image 17. It is possible to save time or operation for acquiring the optical local image or local thermal image again. Also, selection of the optical local image or local thermal image can be linked with reading of the medical image 17 for a corresponding object from the storage device 27 upon displaying the medical image 17. Assuming that the medical image 17 is a sectional image, it may be difficult exactly to recognize an object in the medical image 17 with the appearance, such as a chest or abdomen. However, it is comparatively easy to recognize an object in the optical local image or local thermal image in comparison with sectional images. Accordingly, the redisplay of the medical image 17 can be quickened efficiently by storing the optical local image or local thermal image in the storage device 27 in addition to the medical image 17.

For the purpose of more rapid redisplay of the medical image 17, it is preferable to store relationship information of the optical local image and the local thermal image to the medical image 17. Thus, the medical image 17 can be searched according to the relationship information. It is unnecessary to recognize an object by image analysis of the optical local image and the local thermal image.

In the above embodiments, the images formed by the optical camera 23 or the thermal camera 61 are used for the object specification. However, other methods for the object specification can be used. For example, it is possible to have the body 15 wear a special medical garment for object specification. Storage devices are attached to the medical garment, are positioned at various parts including a head, chest, abdomen, arms and legs, and store an object location (body part recognition information). For example, the storage devices are RFID tags (radio frequency identification tags). An RFID tag at the head stores an object location (body part recognition information) of the head. An RFID tag at the chest stores an object location of the chest. Also, a sensor is disposed on the portable terminal apparatus for reading the RFID tags.

In case the terminal apparatus is placed near to the RFID tag for the head, the sensor in the terminal apparatus reads the object location (body part recognition information) of the head. In case the terminal apparatus is placed near to the RFID tag for the chest, the sensor in the terminal apparatus reads the object location of the chest. It is possible to change a region of the object to be recognized by the terminal apparatus according to changes in the relative position between the body 15 and the terminal apparatus.

Furthermore, an extra display panel can be additionally used for displaying the same image as the display unit 22. In short, multi-screen display is possible by use of a plurality of display panels separate from the display unit 22. Accordingly, the display unit 22 can be mainly used by a doctor and the extra display panel can be specially used by the patient for visual checking. Viewing the medical image 17 can be facilitated both for the doctor and for the patient owing to the separate assignment of the display panels.

In the embodiments, the image storage device for the medical images 17 is the server apparatus 32 in connection with the communication network 31. However, a image storage device can be an internal storage device in the terminal housing 21.

The medical images 17 are CT images according to the above embodiments, but can be MRI images, X-ray images, endoscope images and the like formed by any one of various imaging modalities. A set of the medical images 17 can a combination of images of two or more different imaging modalities, such as CT images and MRI images. Also, a set of the medical images 17 can be a combination of images of two or more different image dates. A set of the medical images 17 can be a combination of images of two or more different imaging modalities and two or more different image dates.

In the above embodiments, examples of the object (body part or anatomical region) are the head, chest, abdomen and the like. However, an object (body part or anatomical region) in the body according to the invention can be a larger object such as an upper half body, and a smaller object such as a shoulder, knee and the like.

In the above embodiments, the portable terminal apparatus is mobile, and connected to the server apparatus by the network wirelessly with the communication interface. However, the portable terminal apparatus of the invention can be an electronic apparatus of a portable size connected to the server apparatus in a wired manner directly by use of a connector without the network. A manual input device can be manipulated to select one of medical images for use, such as a key, joystick, mouse, touch pad and the like.

Furthermore, it is possible to create only a local image without creating a body image. For this structure, at least one reference local image is stored in a memory for each of the various objects or body parts. The local image can be compared with the reference local image to specify an object to be diagnosed.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A portable terminal apparatus for imaging diagnosis comprising:
an object recognition unit (37, 62) for recognizing one object (15a) in a body (15) for imaging diagnosis, so as to output object information of said object;
an image acquisition device (35) for acquiring a medical image (17) from an image storage apparatus (32) with relevancy to said object according to said object information;
a display unit (22) for displaying said acquired medical image.

2. A portable terminal apparatus as defined in claim 1, wherein said object recognition unit includes:
an image sampling device for imaging said object in said body to create a local image; and
an image analyzer for analyzing said local image in image analysis, to specify said object for outputting said object information.

3. A portable terminal apparatus as defined in claim 2, wherein said image sampling device is an optical camera for creating an optical image;
said image analyzer specifies said object by said image analysis of said optical image.

4. A portable terminal apparatus as defined in claim 3, wherein said optical camera creates an optical body image of said body and said local image related to said object in said body;
said image analyzer specifies said object by comparison between said optical body image and said local image.

5. A portable terminal apparatus as defined in claim 4, further comprising a storage device for storing said medical image acquired by said image acquisition device and said local image from said optical camera.

6. A portable terminal apparatus as defined in any one of claims 1 to 5, wherein said medical image is constituted by plural medical images of two or more imaging modalities different from one another, and/or plural medical images of an imaging modality at two or more image dates different from one another.

7. A portable terminal apparatus as defined in any one of claims 1 to 6, wherein said image storage apparatus is an image server connected in communication network connection.

8. A portable terminal apparatus as defined in claim 2, wherein said image sampling device is a thermal camera for creating an image of temperature distribution;
said image analyzer specifies said object by said image analysis of said image of said temperature distribution.

9. A portable terminal apparatus as defined in claim 8, wherein said thermal camera creates a body thermal image of said body and a local thermal image of said temperature distribution related to said object in said body;
said image analyzer specifies said object by comparison between said body thermal image and said local thermal image.

10. A portable terminal apparatus as defined in claim 9, further comprising a storage device for storing said medical image acquired by said image acquisition device and said local thermal image from said thermal camera.

11. A portable terminal apparatus as defined in any one of claims 1 to 10, wherein said medical image is constituted by plural medical images of two or more imaging modalities different from one another, and/or plural medical images of an imaging modality at two or more image dates different from one another.

12. A portable terminal apparatus as defined in any one of claims 1 to 11, wherein said image storage apparatus is an image server connected in communication network connection.

13. A medical image display method for a portable terminal apparatus (10) comprising steps of:
recognizing one object (15a) in a body (15) for imaging diagnosis, so as to output object information of said object;
acquiring a medical image (17) from an image storage apparatus (32) with relevancy to said object according to said object information;
displaying said acquired medical image on said portable terminal apparatus.

14. A medical image display method as defined in claim 13, wherein said object recognizing step includes:
imaging said obj ect in said body with said portable terminal apparatus to create a local image; and
analyzing said local image in image analysis, to specify said object for outputting said object information.
